(19) Europäisches Patentamt
European Patent Office
Office européen des brevets

(11) EP 0 881 489 A1

(12) EUROPEAN PATENT APPLICATION
published in accordance with Art. 158(3) EPC

(43) Date of publication:
02.12.1998 Bulletin 1998/49

(51) Int. Cl.[6]: G01N 33/48, C12Q 1/00

(21) Application number: 96943322.6

(86) International application number:
PCT/JP96/03842

(22) Date of filing: 27.12.1996

(87) International publication number:
WO 97/24616 (10.07.1997 Gazette 1997/30)

(84) Designated Contracting States:
DE

(30) Priority: 29.12.1995 JP 353635/95
08.08.1996 JP 227472/96

(71) Applicant:
WAKO PURE CHEMICAL INDUSTRIES, LTD
Chuo-ku Osaka (JP)

(72) Inventor:
KATO, Fuminori,
Ishihara Sangyo Kaisha, Ltd.
Kusatsu-shi, Shiga 525 (JP)

(74) Representative:
von Kreisler, Alek, Dipl.-Chem. et al
Patentanwälte,
von Kreisler-Selting-Werner,
Bahnhofsvorplatz 1 (Deichmannhaus)
50667 Köln (DE)

(54) METHOD FOR DETERMINING VIABLE CELL COUNT

(57) A method for conveniently and quickly determining the viable cell count and/or survival ratio of cells. This method comprises measuring the fluorescence intensity of a test sample subjected to a treatment with a fluorescent probe for nucleic acid capable of exclusively staining dead cells and the fluorescence intensity of the test sample subjected to not only the treatment with the above-mentioned probe but also another treatment of damaging cell membrane and them comparing these intensities.

Fig. 1 Apparatus for Determining Viable Cell Number

**Description**

TECHNICAL FIELD

The present invention provides a method for conveniently and quickly determining the number of viable cells (i.e., viable cell count or viable cell number) and/or cell viability (i.e., the survival rate of cells) by subjecting cells to a treatment with a fluorescent probe for nucleic acid, capable of exclusively staining dead cells, and also to a treatment of damaging cell membrane, which is applicable for assessment of cytotoxicity in the field of research and development for pharmaceuticals, agricultural chemicals, cosmetics, foods, etc., the field of research for medicine, pharmacy and biology and the field of clinical test and diagnosis.

BACKGROUND ART

Pharmaceuticals, agricultural chemicals, cosmetics, foods, etc. are often assessed for their safety, pharmaceutical and medicinal effect, etc., relying on cytotoxicity. For an index for the cytotoxicity, viable cells are often counted. For detecting and assaying the viable cell number or cell viability, the following prior techniques have been proposed: (1) Trypan Blue staining technique; (2) MTT assay system (Journal of Immunological Methods, Vol. 65, pp. 55-63, 1983); (3) a method described in U.S. Patent No. 5,314,805; (4) a method described in Molecular Medicine, Vol. 32, pp. 1340-1342, 1995; (5) a method described in U.S. Patent No. 5,534,416; etc.

In the technique (1), cells stained with Trypan Blue which stains exclusively dead cells in blue color are visually counted with the naked eye in a field under a microscope. In the system (2), MTT is a substrate for mitochondrial dehydrogenase in cells and the amount of bluish purple formazan which is produced as a product of said enzymatic reaction is assayed by a spectrophotometer. In the method (3), a single sample is made to react with two kinds of fluorescent probes (Calcein AM and ethidium homodimer) and then fluorescence of both viable cells and dead cells is simultaneously measured whereby the viable cell number is determined. Although Calcein AM is a substance which usually generates almost no fluorescence, it is decomposed by the action of esterase in viable cells to produce calcein emitting strong fluorescence. Ethidium homodimer is a fluorescent probe for nucleic acid, having a property capable of passing through damaged cell membranes only, of specifically invading into dead cells only and of binding to nucleic acids. The fluorescence of ethidium binding to nucleic acids is about 40-fold greater than that of free ethidium homodimer. Through those measuring methods, it is possible to easily and quickly determine the cytotoxicity by pharmaceuticals, etc. on multiplates. In the method (4), the cell viability is determined via (a) measuring (i) the fluorescence intensity of test samples treated with propidium iodide which stains dead cell DNA only and (ii) the fluorescence intensity of test samples treated with Hoechst 33258 which stains not only dead cell DNA but also viable cell DNA and (b) comparing both intensities. In the method (5), the cell viability is determined via (a) measuring (i) the fluorescence intensity of test samples treated with a cyanine fluorescent dye (Dye I) (disclosed in U.S. Patent No. 5,436,134) which stains not only dead cell DNA but also viable cell DNA and (ii) the fluorescence intensity of test samples treated with either a cyanine fluorescent dye (disclosed in U.S. Patent No. 5,321,130) or a dyeing agent emitting fluorescence upon acting on viable cells (both agents are called "Dye II") and (b) comparing both intensities

However, in the technique (1), since it is difficult to judge whether they are Trypan Blue stains or not and hard to measure them unless visual observation is applied by the naked eye, it takes much time to complete the measurement. In the system (2) and method (3), both are principally relying on an enzymatic reaction and, therefore, those are apt to be affected by temperature and pH during the measurement, enzymatic reaction time and variations in enzymatic activity among cell strains. Further, in the system (2), not only a centrifugal operation but also an operation of dissolving hardly soluble formazan formed as a result of the enzymatic reaction is necessary. In the method (3), there are disadvantages that (i) blood serum esterase in a cell culture medium decomposes calsein AM and, therefore, the serum is to be removed by washing the cells for several times but, since dead cells are apt to be removed by washing, precise measurement is not achieved unless a system using serum-free cell culture medium is applied; and that (ii) the measurement is complicated because two kinds of fluorescence are measured. In addition, in the methods (4) and (5), there are disadvantages that (i) the operation is complicated because two kinds of fluorescence are to be measured, (ii) when cell viability is measured by treating one sample with two kinds of fluorescent dyes, the fluorescent dye used later is badly affected by that used earlier, and (iii) in order to determine the cell viability (survival ratio), it is necessary to prepare a calibration curve for each measurement.

DISCLOSURE OF THE INVENTION

The present inventors have conducted various investigations in order to provide a method for determining viable cell number which satisfies the conditions including:

(1) viable cell numbers in a large number of samples can be precisely determined in a convenient and quick manner;

(2) detectable for even test samples where blood serum coexists in the cell culture medium; (3) free of troublesome operations to the sample, such as cell washing, centrifugal separation of cells, dissolution of crystals, etc.; (4) the measured result is rarely affected by variations depending on temperature for the measurement, pH for the measurement, time for reacting with a reagent, kinds of cell, etc. As a result thereof, they have succeeded in accomplishing the present invention.

The present invention relates to:

(I). a method for determining the number of viable cells (i.e., viable cell number) and/or cell viability (i.e., the survival ratio of cells), which comprises

(i) measuring the fluorescence intensity of a test sample subjected to a treatment with a fluorescent probe (or dye) for nucleic acid, said probe being capable of exclusively staining dead cells,
(ii) measuring the fluorescence intensity of a test sample subjected to (a) a treatment with said fluorescent probe for nucleic acid and (b) a treatment of damaging cell membranes, and
(iii) comparing both fluorescence intensities; and

(II). a kit for the measurement of viable cell number and/or cell viability by the following steps:

(i) measuring the fluorescence intensity of a test sample subjected to a treatment with a fluorescent probe for nucleic acid, capable of exclusively staining dead cells,
(ii) measuring the fluorescence intensity of a test sample subjected to treatments with (a) said fluorescent probe for nucleic acid and (b) an agent capable of damaging cell membranes, and
(iii) comparing both fluorescence intensities, which comprises

(A) said fluorescent probe for nucleic acid, capable of exclusively staining dead cells, and
(B) said agent capable of damaging cell membranes.

One aspect of the present invention is:

(1). a method for determining viable cell number and/or cell viability, which comprises

(i) measuring the fluorescence intensity of a test sample subjected to a treatment with a fluorescent probe for nucleic acid, capable of exclusively staining dead cells,
(ii) measuring the fluorescence intensity of a test sample subjected to not only (a) a treatment with said fluorescent probe for nucleic acid but also (b) a treatment of damaging cell membranes, and
(iii) comparing both fluorescence intensities;

(2). the method according to the above (1) wherein

(i) the test sample is treated with a fluorescent probe for nucleic acid, capable of exclusively staining dead cells, and the resulted fluorescence intensity is measured, and
(ii) said test sample is then subjected to a treatment for damaging cell membranes, so that cells would become extinct, thereby intensifying the fluorescence thereof and the resultant fluorescence intensity thereof is measured;

(3). the method according to the above (1) wherein

(i) the test sample is treated with a fluorescent probe for nucleic acid, capable of exclusively staining dead cells, and the resulted fluorescence intensity of said test sample is measured, and
(ii) another test sample is subjected to not only (a) a treatment with said fluorescent probe for nucleic acid but also (b) a treatment of damaging cell membrane, and the resulted fluorescence intensity thereof is measured;

(4). the method according to any of the above (1) to (3) wherein the fluorescent probe for nucleic acid is a cationic fluorescent stain (or dye) for nucleic acid;
(5). the method according to any of the above (1) to (4) wherein the cell membrane is damaged with an agent capable of damaging cell membranes;

(6). the method according to the above (5) wherein the agent capable of damaging cell membranes is a surface-active agent;

(7). the method according to any of the above (1) to (6) wherein each operation in a series of the following steps is conducted by an automated apparatus or system:

    (i) measuring the fluorescence intensity of a test sample subjected to a treatment with a fluorescent probe for nucleic acid, capable of exclusively staining dead cells,

    (ii) measuring the fluorescence intensity of a test sample subjected to not only (a) a treatment with said fluorescent probe for nucleic acid but also (b) a treatment of damaging cell membranes, and

    (iii) comparing both fluorescence intensities;

(8). a kit for determining viable cell number and/or cell viability by the following steps:

    (i) measuring the fluorescence intensity of a test sample subjected to a treatment with a fluorescent probe for nucleic acid, capable of exclusively staining dead cells,

    (ii) measuring the fluorescence intensity of a test sample subjected to treatments with (a) said fluorescent probe for nucleic acid and (b) an agent capable of damaging cell membranes, and

    (iii) comparing both fluorescence intensities, which comprises

        (A) said fluorescent probe for nucleic acid, capable of exclusively staining dead cells, and

        (B) said agent capable of damaging cell membranes.

    Another aspect of the present invention is:

(9). the method according to any of the above (1) to (3) wherein the fluorescent probe for nucleic acid is ethidium homodimer or a cyanine fluorescent dye capable of exclusively staining dead cells;

(10). the method according to any of the above (1) to (3) wherein the fluorescent probe for nucleic acid is a cyanine fluorescent dye capable of exclusively staining dead cells;

(11). the method according to any of the above (5), (6), (9) and (10) wherein the agent capable of damaging cell membranes is a polyoxyethylene nonionic surface-active agent or alcohol nonionic surface-active agent;

(12). the method according to any of the above (5), (6), (9) and (10) wherein the agent capable of damaging cell membranes is at least one surface-active agent selected from the group consisting of Triton X-100 (trade name), Triton X-114 (trade name) and Nonidet P-40 (trade name);

(13). the kit according to the above (8) wherein the fluorescent probe for nucleic acid is a cationic fluorescent stain for nucleic acid;

(14). the kit according to the above (8) wherein the fluorescent probe for nucleic acid is ethidium homodimer or a a cyanine fluorescent dye capable of exclusively staining dead cells;

(15). the kit according to the above (8) wherein the fluorescent probe for nucleic acid is a cyanine fluorescent dye capable of exclusively staining dead cells;

(16). the kit according to any of the above (8), (13), (14) and (15) wherein the agent capable of damaging cell membranes is a polyoxyethylene nonionic surface-active agent or alcohol nonionic surface-active agent; and

(17). the kit according to any of the above (8), (13), (14) and (15) wherein the agent capable of damaging cell membranes is at least one surface-active agent selected from the group consisting of Triton X-100 (trade name), Triton X-114 (trade name) and Nonidet P-40 (trade name).

    Still another aspect of the present invention is:

(18). an apparatus (or system) for determining viable cell number and/or cell viability, which comprises at least

    (a) a means for supplying a test sample with a fluorescent probe for nucleic acid, capable of exclusively staining dead cells,

    (b) a means for measuring the fluorescence intensity of the test sample which is treated with said fluorescent probe for nucleic acid,

    (c) a means enabling us to damage cell membrane,

    (d) a means for measuring the fluorescence intensity of the test sample which is subjected to not only (A) a treatment with said fluorescent probe for nucleic acid but also (B) a treatment of damaging cell membrane, and

    (e) a means for comparing the fluorescence intensity (b) with the fluorescence intensity (d);

(19). the apparatus according to the above (18) wherein (i) the test sample to be measured is treated with a fluorescent probe for nucleic acid which stains dead cells only and the intensity for the emitted fluorescence resulted thereby is measured and then (ii) said test sample is subjected to a treatment of damaging the cell membrane thereof, so that the cells are destined to become extinct and the intensity for the fluorescence intensified thereby is measured;

(20). the apparatus according to the above (18) or (19) wherein the above-mentioned (i) and (ii) are conducted in an automated manner;

(21). the apparatus according to the above (20) wherein each treatment is conducted in an automatically controlled manner by the use of a microprocessor according to a program which is installed in advance therein;

(22). the apparatus according to any of the above (18) to (21) wherein said apparatus is equipped with

> (a) a device for holding an agent capable of damaging cell membranes,
> (b) a device for holding a fluorescent probe for nucleic acid capable of exclusively staining dead cells,
> (c) an injector for drug capable of supplying the test sample with the agent from the above-mentioned device (a) or (b),
> (d) a container, such as a microplate or cuvette for test equipped with a mixer, capable of holding the test sample for assay therein, and
> (e) a spectrofluorophotometer; and

(23). the apparatus according to (18) wherein the apparatus is equipped with

> (a) a device capable of, for a single test sample,
>
>> (i) measuring the intensity for the fluorescence emitted by the test sample which is subjected to a treatment with a fluorescent probe for nucleic acid, capable of exclusively staining dead cells, and
>> (ii) then measuring the intensity for the fluorescence intensified by a treatment of damaging cell membranes wherein the cells would become extinct, or
>
> (b) a device capable of
>
>> (i) measuring the intensity for the fluorescence emitted by a test sample subjected to a treatment with the fluorescent probe for nucleic acid, and
>> (ii) measuring the intensity for the fluorescence emitted by another test sample subjected to (A) a treatment with the fluorescent probe for nucleic acid and (B) a treatment of damaging cell membranes.

Still another aspect of the present invention is:

(24). a method for evaluating the toxicity of a substance, which comprises

> (a) treating cells with the substance requested to be evaluated for toxicity,
> (b) treating the resulting test sample with a fluorescent probe for nucleic acid, capable of exclusively staining dead cells, followed by measuring the intensity for the fluorescence emitted by the resultant test sample,
> (c) subjecting the resulting test sample to a treatment with the fluorescent probe for nucleic acid as well as a treatment of damaging cell membranes, followed by measuring the intensity for the fluorescence emitted by the resultant test sample,
> (d) determining viable cell number and/or cell viability via comparing both fluorescence intensities;

(25). the method according to the above (24) wherein

> (i) cells are treated with a substance which is to evaluated for toxicity,
> (ii) the resulting test sample to be measured is treated with a fluorescent probe for nucleic acid, capable of exclusively staining dead cells, followed by measurement of the fluorescence intensity of the resulting test sample and
> (iii) this test sample is subjected to a treatment of damaging cell membrane, so that the cells would become extinct, followed by measurement of the intensity for the fluorescence intensified thereby; and

(26). the method according to the above (24) wherein

> (i) cells are treated with a substance which is to evaluated for toxicity,

(ii) one test sample to be measured is treated with a fluorescent probe for nucleic acid, capable of exclusively staining dead cells, followed by measurement of the fluorescence intensity of the resulting test sample and

(iii) another test sample is subjected to (a) a treatment with the fluorescent probe for nucleic acid as well as (b) a treatment of damaging cell membrane, followed by measurement of the intensity for the fluorescence emitted by the resultant test sample.

BRIEF DESCRIPTION OF DRAWINGS

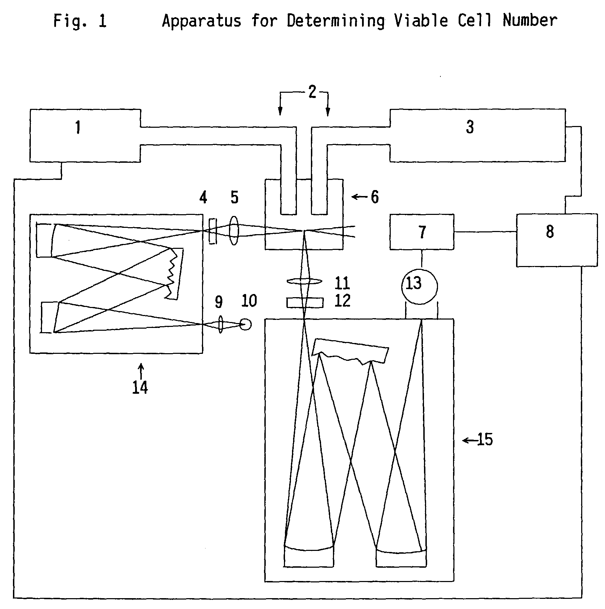

Fig. 1 is an embodiment of an apparatus (or system) for determining viable cell number and/or cell viability according to the present invention. In this apparatus, the present invention can be conducted by counting dead cells on a single test sample, followed by counting total cells.

| 1 | agent capable of damaging cell membranes |
| 2 | injector for drug |
| 3 | dye (for nucleic acid) capable of staining dead cells |
| 10 | light source |
| 6 | cuvette (for test sample) equipped with a mixer |
| 5, 9 and 11 | lens |
| 7 | transducer capable of transforming analog signal into digital |
| 4 and 12 | polarizing plate |
| 13 | photomultiplier |
| 8 | control system |
| 14 | excitation spectroscope |
| 15 | fluorescence spectrophotometer |

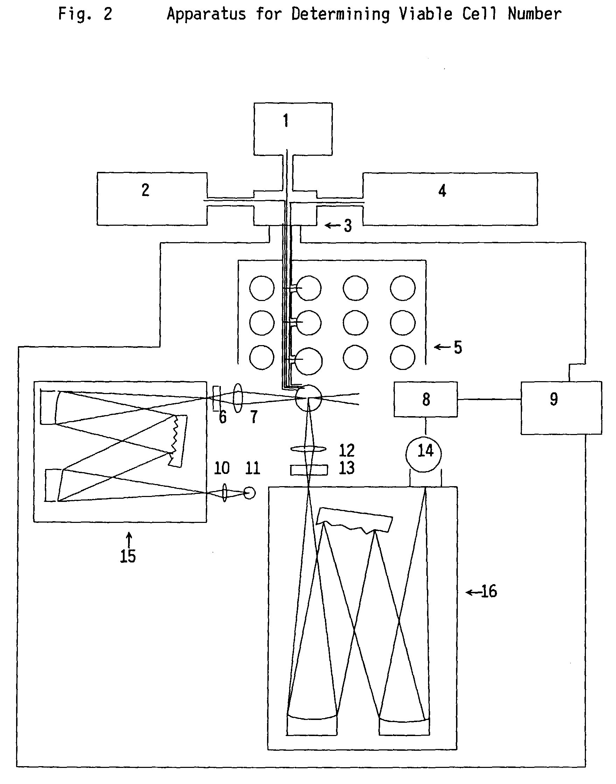

Fig. 2 is another embodiment of an apparatus (or system) for determining viable cell number and/or cell viability according to the present invention. In this system, the present invention can be conducted by preparing several test samples to be measured under a single condition, counting dead cells for one half of said test samples by the above-mentioned method while another half thereof is subjected to a treatment with fluorescent probe for nucleic acid and also to a treatment of damaging cell membrane, followed by, similarly to the case of the above method, measuring the number of dead cells for the test samples for counting dead cells and determining the total number of cells.

| 1 | diluted cell suspension |
| 2 | agent capable of damaging cell membrane |
| 3 | dispenser |
| 4 | dye (for nucleic acid) capable of staining dead cells |
| 5 | 96-well microplate equipped with a plate mixer |
| 6 and 13 | polarizing plate |
| 7, 10 and 12 | lens |
| 8 | transducer capable of transforming analog signal into digital |
| 9 | control system |
| 11 | light source |
| 15 | excitation spectroscope |
| 16 | fluorescence spectrophotometer |
| 14 | photomultiplier |

Fig. 3 shows a relation between the number of floating cells and the fluorescence intensity measured by the present invention. It is shown that there is a proportional relationship between the number of cells and the fluorescence intensity.

Fig. 4 shows a relation between the number of adhesive cells and the fluorescence intensity measured by the present invention. It is shown that there is a proportional relationship between the number of cells and the fluorescence intensity.

BEST MODE FOR CONDUCTING THE INVENTION

The test sample to be measured to which the present invention is applicable may be any cell so far as it has no cell wall. Examples of such cells are animal cells, a part of microbial cells, protoplasts derived from microorganisms and plants wherein cell membranes are removed and the like. Any animal cells are used as test samples. Examples thereof include animal body fluid cells (blood cells, lymphocytes, etc.) and animal cancer cells (animal tumor cells). Those samples may be anything so far as they are under an environment where the cell can be alive. Examples thereof are cells in a cultured medium. When pharmaceuticals, agricultural chemicals, cosmetics, foods, etc. having cytotoxicity are made coexisting in the living environment of those samples, a part or all of the cells in the sample become dead cells. When viable cell numbers are determined under such an environment, assessment of the cytotoxicity can be achieved.

The fluorescent probe for nucleic acid capable of exclusively staining dead cells (or fluorescent probe for nucleic acid which stains dead cells only) means the agent having the following characteristic features:

(1) it passes through damaged cell membrane but does not pass through undamaged cell membrane;
(2) it emits weak fluorescence or hardly emits the fluorescence in a state where it is not bonded to nucleic acid; and
(3) it emits strong fluorescence when bonded to nucleic acid.

Embodiments of the fluorescent probe for nucleic acid may include cationic fluorescent stains (dyes) for nucleic acid, such as ethidium homodimer and a cyanine fluorescent dye; ethidium halides such as ethidium bromide; propidium halides such as propidium iodide; and the like. It is preferred that ethidium homodimer or a cyanine fluorescent dye capable of exclusively staining dead cells is used in the present invention. It is particularly preferred that a cyanine fluorescent dye which can exclusively stain dead cells is used herein. The cyanine fluorescent dyes capable of exclusively staining dead cells include those described in U.S. Patent No. 5,321,130. Examples of the cyanine fluorescent dye useful in the present invention are BOBO-1 Iodide, BOBO-3 Iodide, BO-PRO-1 Iodide, BO-PRO-3 Iodide, POPO-1 Iodide, POPO-3 Iodide, PO-PRO-1 Iodide, PO-PRO-3 Iodide, TOTO-1 Iodide, TOTO-3 Iodide, TO-PRO-1 Iodide, TO-PRO-3 Iodide, YOYO-1 Iodide, YOYO-3 Iodide, YO-PRO-1 Iodide, YO-PRO-3 Iodide (all of them are trade names; manufactured by Molecular Probes, U.S.A.) and the like. Fluorescent emission wavelength varies depending upon variations in measuring conditions such as kinds of cells to be assayed and species of targets which are to be assessed for cytotoxicity wherein said targets may include pharmaceuticals, agricultural chemicals, cosmetics and foods. Since each fluorescent probe for nucleic acid has its own intrinsic excitation wavelength and fluorescent emission wavelength, an appropriate fluorescent probe for nucleic acid may be suitably selected and used depending upon the measuring condition. Further, there are some cases where the fluorescent probe for nucleic acid itself has its own fluorescence in addition to the culture medium and the target to be assessed for cytotoxicity, it is necessary to measure the fluorescence intensity (Fb) of the background derived thereby with a fluorometer in advance.

The above-mentioned fluorescent probe for nucleic acid has a property that it passes through damaged cell membranes or the cell membrane of dead cells but is unable to pass through undamaged cell membranes or the cell membrane of viable cells. Therefore, when this fluorescent probe is applied to a test sample in which both viable and dead cells are coexisting, the fluorescent probe selectively permeates into the dead cells and bonds with nucleic acids to emit a strong fluorescence. When this fluorescence intensity (Fd') is measured by a fluorometer, the dead cell numbers can be determined. To be more specific, fluorescence intensity (Fd) corresponding to the number of dead cells is calculated from the following equation:

$$Fd = Fd' - Fb$$

From the resulting value, the number of dead cells can be determined by means of a calibration curve or the like. It is also possible to directly determine the cell viability without calculating the number of dead cells.

The methods for damaging cell membrane for the test sample to be measured include a method wherein an agent capable of damaging cell membrane (or agent for damaging cell membrane) is applied, a method wherein cell membranes are damaged (or injured) by a physical means such as application of ultrasonic wave, etc.

The agent capable of damaging cell membranes (i.e., agent for damaging cell membranes) may be any agent as long as it damages or injures the cell membrane of viable cells with the result that all the viable cells in the system would (or are destined to) become extinct (or would be killed). Examples of the agent capable of damaging cell membranes are surface-active agents; acids such as hydrochloric acid and sulfuric acid; bases such as sodium hydroxide and potassium hydroxide; etc. In order to distinctly conduct the measurement of the fluorescence intensity which will be mentioned later, it is preferred to use a surface-active agent among the above-mentioned ones. More specific examples thereof are polyoxyethylene ether nonionic surface-active agents such as Triton X-100, Triton X-114, Triton X-305, Triton X-405, Brij-35, Brij-56 and Brij-58 (all of them are trade names; manufactured by Pierce Chemical); ester nonionic surface-active agents such as Tween 20, Tween 80 and Span 20 (all of them are trade names; manufactured by Pierce Chemical); alcohol type nonionic surface-active agents such as Nonidet P-40 (trade name; manufactured by Pierce Chemical); cholic acid type nonionic surface-active agents such as CHAPS, CHAPSO, BIGCHAP and DEOXY-BIGCHAP (all of them are trade names; manufactured by Pierce Chemical); glycoside nonionic surface-active agents such as hexyl-β-D-glucopyranoside, octyl-β-D-glucopyranoside, octyl-β-glucoside, octyl-β-thioglucopyranoside and octylglucopyranoside; anionic surface-active agents such as N-lauroyl sarcosine, sodium N-lauroyl sarcosine, sodium laurylsulfate, lithium laurylsulfate and sodium dodecylsulfate; and saponins such as saponin, Panax ginseng saponins, glycyrrhizin, glycyrrhizic acid and Bupleurum saponins. Among them, polyoxyethylene ether nonionic surface-active agents or alcohol type nonionic surface-active agents are preferably used. Particularly, Triton X-100 (trade name), Triton X-114 (trade name) or Nonidet P-40 (trade name) is preferred. Those agents for damaging the cell membrane may be used either alone or jointly in a mixed form.

In case where the agent capable of damaging cell membranes is applied, a solution of said agent with a desired concentration can be applied by dropping into a stationary culture medium containing cells or into a stirred culture medium. Alternatively, a culture medium containing cells, etc. can be added to a liquid containing said agent (capable of damaging cell membranes) in a predetermined concentration either gently or with stirring to form a mixture. In that case, addition may be conducted with a tool such as pipette, injector (syringe), pipetter, dispenser, and spuit. In some cases, it is possible that the cells may be temporarily contacted with the agent capable of damaging cell membranes. Thus, for example, they may be just dipped in a solution containing said agent (capable of damaging cell membranes). Preferably, such treatments can be conducted with an automated device equipped with microprocessors by adding a certain amount of a solution containing said agent to a culture medium in accordance with a predetermined procedure. The device used therefor may be suitably selected from those which are known in the art.

When cell membranes are damaged by a physical means, not only cell membranes but also DNA, etc. in the cells may be damaged in some cases and, therefore, the treating condition is to be carefully selected if necessary. For example, when ultrasonication is applied, it is possible to treat the test sample by dipping a stirring rod thereinto or by placing it in a cup-like container equipped with a stirring rod. It is also possible to use a continuously operable device. Such operable devices used therefor may be suitably selected from those which are known in the art. It is further possible to use an osmotic pressure shock method, a freezing and thawing method, etc. for damaging the cell membrane.

When it is difficult to determine the cell viability directly from the acquired data, a new calibration curve is prepared, if necessary, for each of the treating conditions so that the measured value is corrected.

When the cell membrane of viable cells present in the test sample after the measurement of dead cells is subjected to a damaging treatment to make the cells extinct, the fluorescent probe for nucleic acid also invades into the cell which was once viable and is allowed to bond with nucleic acids and to emit fluorescence. Accordingly, the result is that the test sample has now the intensified fluorescence which gains by an amount of the fluorescence emitted by the cells which were once viable. When the intensified fluorescence intensity (Ft') thereof is measured by a fluorometer, it is now possible to quantitatively determine the total number of cells (i.e., total cell number) existing in the test sample and then viable cell number can be determined by subtracting the dead cell number from the total cell number. To be more specific,

$$Ft = Ft' - Fb' \text{ and}$$

$$Fl = Ft - Fd = (Ft' - Fb') - (Fd' - Fb)$$

are used for calculating the fluorescence intensity (Ft) corresponding to the total cell number and that (Fl) corresponding to the viable cell number. From those data, the total number of cells and the number of living cells, i.e. the number of viable cells, can be quantitatively determined by means of calibration curves, etc.

Fb' is a background fluorescence intensity from the agent capable of damaging cell membranes, the fluorescent probe for nucleic acid, the culture medium and the target to be evaluated for cytotoxicity. When a surface-active agent is applied for damaging the cell membrane, there are many cases where Fb = Fb' and, in that case, Fl can be calculated by:

$$Fl = Ft' - Fd'$$

Several test samples under the same condition (i.e., in a single condition) are prepared and one half of the test samples is subjected to a determination for the above-mentioned dead cell numbers by the aforementioned technique while another half thereof is subjected to a treatment with a fluorescent probe for nucleic acid and a treatment of damaging the cell membrane whereby the total cell numbers are determined by the same manner as in the case of the aforementioned method. When the dead cell numbers are subtracted from the total cell numbers calculated as such, the viable cell numbers can be determined as well. In this method, it is possible to simultaneously conduct a step of determining the dead cell number and another step of determining the total cell number and, therefore, an efficient determination can be conducted. However, there is a possibility of resulting in the problems that it is necessary to conduct the measurement for many test samples and that the measurement error is big. On the other hand, when dead cell numbers are first determined and total cell numbers are then determined for the same sample, the time interval between the former and the latter measuring steps causes a problem. Such a time interval may vary depending upon various conditions such as kinds of cell lines to be assayed, fluorescent probes for nucleic acid, agents capable of damaging cell membranes, etc. and a short time is recommended, preferably within two hours, or more preferably within one hour. Accordingly, it is preferred that various conditions such as kinds of cell lines to be assayed, fluorescent probes for nucleic acid, agents capable of damaging cell membranes, etc. are taken into consideration to select the most suitable determination under the given condition.

The cell viability can be calculated from the total cell number and viable cell number data obtained by the assay. When the dead cell numbers are presumed to be nearly zero, then Fl ≒ Ft. Therefore, if fluorescence intensity is measured in such a manner that both a treatment with the fluorescent probe for nucleic acid and a treatment with the agent capable of damaging cell membranes are simultaneously conducted, it is possible to determine the total cell number or the viable cell number only by one step of fluorescence measurement. In the case of pharmaceuticals, agricultural chemicals, cosmetics, foods, etc., they are frequently assessed for their cytotoxicity, relying on data in terms of viable cell number and cell viability. For example, there is a case where the concentration of drugs, etc. making 50% cells dead (i.e. $IC_{50}$) is calculated from the viable cell number data. In accordance with the technique of the present invention, such a cytotoxicity can be conveniently and efficiently assessed.

In accordance with the present invention, it is possible to directly determine the cell viability without counting the viable cell number.

The method according to the present invention can be conducted using a measuring instrument having a function of outputting viable cell number and/or cell viability data by means of combination and systematization of each operation in a series of the following steps:

first step: (i) measuring the fluorescence intensity of a test sample subjected to a treatment with a fluorescent probe for nucleic acid, capable of exclusively staining dead cells, and (ii) measuring the fluorescence intensity of a test sample subjected to not only (a) a treatment with said fluorescent probe for nucleic acid but also (b) a treatment of damaging cell membranes (this step includes each treatment which is conducted prior to each measurement), and second step: comparing both fluorescence intensities.

In said measuring instrument, it is preferred to conduct the present invention to utilize a system in which both a fluorescent probe for nucleic acid capable of exclusively staining dead cells and an agent capable of damaging cell membranes are set for conducting each treatment applied prior to each measurement in the first step of said system. In such a measuring instrument, it is possible to apply a kit for determining the viable cell number and/or a kit for determining the cell viability and, accordingly, the determination of viable cell numbers and/or cell viability according to the present invention can be conducted efficiently. Embodiments of the apparatus (or system) which conducts the assay according to the present invention in an automated manner include those as shown in Figs. 1 and 2. In said system, a term reading "apparatus for determining viable cell number" is used but it may also be understood as "apparatus for determining cell viability" or "apparatus for determining viable cell number and/or cell viability". In the present specification and claims, said apparatus (or system) is to be understood in a broad sense.

Fig. 1 is an embodiment of an apparatus for determining the viable cell numbers wherein the number of dead cells is first counted and then the total number of cells is counted for a single test sample in accordance with the present invention. As such, said apparatus comprises a spectrofluorophotometer installed therein. Said apparatus comprises a cuvette (for test sample) (6) equipped with a mixer, an excitation spectroscope (14), a fluorescence spectrophotometer (15), a light source (10), lenses (5, 9 and 11), polarizing plates (4 and 12) and a photomultiplier (13). By referring to Fig. 1, (1) is an agent capable of damaging the cell membrane while (3) is a fluorescent probe for nucleic acid, capable of exclusively staining dead cells, and each of the reagents is set either in a tank or in a detachable container in the apparatus. The reagent which is set in such a tank or detachable container can be injected using an injector (2) into the cuvette (6) equipped with a mixer. The apparatus is suitably equipped with a valve and a pump (not shown) for control. If necessary, it may also be optionally equipped with a washing pump, a discharging pump, a directional control valve, an air pump for stirring, a nozzle, etc. In the drawing, (7) is a transducer for transforming analog signals to digital ones and (8) is a control system. The cuvette (6) may be sequentially moved upon necessity whereby plural cuvettes may be automatically measured. It is also possible that the cuvette for test sample is kept in a constant-temperature bath. It is further possible that the cuvette for test sample (6) is immobilized while the injector and the spectrofluorophotometer are sequentially moved if necessary to conduct an automatic measurement.

Fig. 2 illustrates an embodiment of an apparatus for determining the viable cell number for the case where several test samples under the same condition are prepared, one half of the test samples is subjected to a measurement for the aforementioned dead cell number by the aforementioned technique while another half thereof is subjected to a treatment with a fluorescent probe for nucleic acid and a treatment for damaging the cell membrane whereby the dead cell number is measured for the test sample to be assayed for the dead cell number in the same manner as in the aforementioned technique followed by measurement of the total cell number. As such, said apparatus comprises a spectrofluorophotometer incorporated therein. Said apparatus comprises a 96-well microplate (5) equipped with a plate mixer, a excitation spectroscope (15), a fluorescence spectrophotometer (16), a light source (11), lenses (7, 10 and 12), polarizing plates (6 and 13) and a photomultiplier (14). In the drawing, (1) is a diluted suspension of cells, which is set in a tank or detachable container in the apparatus.

In order to supply each well with a homogeneous suspension in terms of cell density from the tank, a mixer is installed either in the tank or in the detachable container. (2) is an agent capable of damaging cell membrane and (4) is a dye for nucleic acid, capable of exclusively staining dead cells, and both said reagents are each set in a tank or detachable container in the apparatus. The suspension or reagent set in such a tank or detachable container can be distributed with a dispenser (3) into each well on a 96-well microplate (5) equipped with a plate mixer. A disposable chip or nozzle is installed at the top end portion of the dispenser (3). Such a disposable chip or nozzle may be optionally exchanged or washed if necessary. The top end of the dispenser (3) may be branched whereby it is capable of distributing the reagent to two or more sample wells simultaneously. In the drawing, (8) is a transducer for transforming analog signals to digital ones and (9) is a control system. Said microplates (5) may be made sequentially and continuously movable if necessary, so that automatic measurement for two or more microplates is possible. It is also possible that said microplates are fixed while the dispenser (3) and the spectrofluorophotometer are sequentially and continuously movable, so that automatic measurement can be conducted.

In case where the present invention is conducted in such a manner that dead cell numbers are measured and then total cell numbers are measured for the same sample, it is efficient to conduct the measurement using an apparatus as shown in Fig. 1.

Described hereinbelow is an example of "how to use" such an apparatus.

(I) A test sample such as a cell sample in a culture medium is set in a cuvette for test sample (6), equipped with a mixer, followed by stirring. A fluorescent probe for nucleic acid capable of exclusively staining dead cells (1) and an agent capable of damaging cell membrane (3) are set in a tank in the apparatus in advance.

(II) The fluorescent probe for nucleic acid capable of exclusively staining dead cells (1) is distributed with a dispenser (2) into each cuvette (6) (for test sample) wherein the test sample is set. As a result, the test sample is stained. Thereafter, intensity of fluorescence emitted therefrom is measured by a spectrofluorophotometer. The resultant measurement data are processed by converting analog signal forms to digital ones and transmitted to a control system (8). The amount of the fluorescent probe (1) (for nucleic acid, capable of exclusively staining dead cells) used is automatically controlled under being linked with a control system (8) for measuring the fluorescence intensity.

(III) The agent (1) (of damaging cell membranes) is distributed with the dispenser (2) into the cuvette (6) (for test sample) after completion of the measurement process (II) with the result that all cells in the test sample are made dead. Thereafter, intensity of the fluorescence emitted therefrom is measured by a spectrofluorophotometer. The resultant measurement data are processed by converting analog signal forms into digital ones and transmitted to the control system (8). The cell viability is determined by comparing with the data obtained in the measurement process (II). If necessary, it is also possible that the above-mentioned measurement is conducted for several samples wherein the cell number is known and appropriate to prepare a calibration curve for this apparatus, so that the viable cell number, etc. is determined for the samples where the cell number is unknown.

It is efficient to conduct the measurements using an apparatus as shown in Fig. 2 in the case where several test samples are prepared under a single condition and one half of the test samples is subjected to a dead cell number measurement by the above-mentioned method while another half is subjected to a treatment with a fluorescent probe for nucleic acid and to a treatment of damaging cell membrane whereby the total cell number is calculated in the same manner as in the above-mentioned method.

Described hereinbelow is also an example of "how to use" such an apparatus.

(I) For preparing test samples under the same condition, the test sample is distributed as a diluted cell suspension (1) with a dispenser (3) into each well on a 96-well microplate (5) equipped with a plate mixer. Thereafter, the microplate well is agitated with a plate mixer. A fluorescent probe (2) (for nucleic acid capable of exclusively staining dead cells) and an agent (4) (capable of damaging cell membrane) are set in a tank in the apparatus in advance.

(II) The fluorescent probe for nucleic acid (2) (capable of exclusively staining dead cells) is distributed with a dispenser into a 96-well microplate (5) wherein the test sample is set. As a result, the test sample is stained. Thereafter, intensity of the fluorescence emitted from the sample in each well is measured by a spectrofluorophotometer. The obtained measurement data are processed by converting analog signal forms to digital ones and transmitted to a control system (9). The amount of the fluorescent probe for nucleic acid (4) (capable of exclusively staining dead cells) is automatically controlled in combination with the control system (9) for the fluorescence intensity measurement. Different dispensing nozzles for each reagent are installed in the dispenser so that the reagent can be distributed into plural wells by a single operation.

(III) Each of the agent (2) (capable of damaging cell membrane) and the fluorescent probe (4) (for nucleic acid capable of exclusively staining dead cells) is distributed with a dispenser into a 96-well microplate (5) where the test samples under the same condition as that used in the measurement (II) are set. As a result, all cells in the test sample are made extinct and simultaneously stained. Thereafter, intensity of the fluorescence emitted from the test sample in each well is measured by a spectrofluorophotometer. The obtained measurement data are processed by converting analog signal forms to digital ones and transmitted to the control system (9). Each amount of the agent (2) (capable of damaging cell membrane) and the fluorescent probe (4) (for nucleic acid capable of exclusively staining dead cells) is automatically controlled in combination with the control system (9) for the fluorescence intensity measurement. The measured analog signal data are transformed into digital ones and communicated to the control system (9) and cell viability is determined by comparing with the data obtained in the measurement process (II). If necessary, it is also possible that the above-mentioned measurement is conducted for several samples wherein the cell number is known and appropriate to prepare a calibration curve for this apparatus, so that the viable cell number, etc. is determined for the samples where the cell number is unknown.

Thus, the present invention provides an apparatus for determining viable cell number and/or cell viability, characterized in that,
said apparatus is equipped with at least:

(a) a means for supplying a test sample with a fluorescent probe for nucleic acid (capable of exclusively staining dead cells),
(b) a means for measuring the fluorescence intensity of the test sample treated with said fluorescent probe for nucleic acid,
(c) a means enabling us to damage the cell membrane, and
(d) a means for measuring the fluorescence intensity of the test sample subjected to not only a treatment with a fluorescent probe for nucleic acid (capable of exclusively staining dead cells) but also a treatment of damaging the membrane and
said apparatus is further equipped with a means for comparing both intensities of the above (b) and (d) for each of the fluorescence intensities as measured.

Each of those constituting elements in said apparatus may be used by selecting from those which have been known in the art, including automated apparatus for immunoassays and biochemical assays or may be used after suitable modifications thereto if necessary.
Preferred apparatus includes a system in which a computer controlled by a program, such as a microcomputer, regulates one or plural means, or all of the above-mentioned means, i.e.,

(a) a means for supplying a test sample with a fluorescent probe for nucleic acid (capable of exclusively staining dead cells),
(b) a means for measuring the fluorescence intensity of the test sample treated with said fluorescent probe for nucleic acid,
(c) a means enabling us to damage the cell membrane, and
(d) a means for measuring the fluorescence intensity of the test sample subjected to not only a treatment with a fluorescent probe for nucleic acid (capable of exclusively staining dead cells) but also a treatment of damaging the membrane and
a means for comparing both intensities of the above (b) and (d) for each of the fluorescence intensities as measured.

In the method for determining viable cell number and/or cell viability and also the kit for determining viable cell number and/or cell viability in accordance with the present invention, it is possible to jointly use each of various fluorescent probes for nucleic acid (capable of exclusively staining dead cells) as aforementioned, in combination with each of various agents (capable of damaging cell membranes) as aforementioned. It is preferred that the aforementioned fluorescent probe for nucleic acid (capable of exclusively staining dead cells) is used in combination with any of the aforementioned various surface-active agents.
It is also possible to use a mixture prepared by mixing the aforementioned fluorescent probe for nucleic acid (capable of exclusively staining dead cells) with any of the aforementioned various surface-active agents, if necessary in advance.
A preferred embodiment of the above-mentioned kit (for measuring the viable cell number and/or cell viability) applicable to and suitable for an apparatus for measuring the viable cell number (or apparatus for measuring the viable cell number and/or cell viability) as shown in Figs. 1 or Fig. 2 is a form wherein each of the fluorescent probes for nucleic acid (capable of exclusively staining dead cells) and the agents (capable of damaging cell membranes) is in advance charged in a detachable container for said apparatus. In that case, said fluorescent probes and said agents (capable of damaging cell membranes) are each charged in different detachable containers but, if necessary, both may be charged in the same detachable container.

EXAMPLES

Described below are examples of the present invention which are provided only for illustrative purposes, and not to limit the scope of the present invention. It is to be understood that the present invention is not limited to those examples but includes numerous embodiments.

Example 1 (Determination of Cell Viability)

(1) Method and Result

① MOLT-4 cells (human acute lymphoblastic leukemia cells; received from Microbiological Laboratory of Osaka University, Japan) were cultured on a Petri dish at 37°C under a 100% humidified atmosphere of air containing 5% carbon dioxide. The culture medium used was RPMI medium (No. 31800-071, manufactured by Gibco), supplemented with 10% fetal calf serum (hereinafter, referred to as "culture medium").
② When a cell density becomes high in the culture medium, samples on a Petri dish were used as test samples for determining the cell viability. The test samples include (i) a sample on a Petri dish where the medium was appropriately changed (sample under good culture condition) and (ii) two samples which were incubated on a Petri dish without changing the medium (samples (1) and (2) under bad culture condition).
③ Each test sample was collected from the Petri dish, placed in a cuvette for test sample and subjected to the measurement described hereinbelow while agitating with a stirring rod.
Fluorescence intensity was measured by a spectrofluorophotometer (Hitachi type F-4000, manufactured by Hitachi, Ltd., Japan) at excitation wavelength: 420 nm and emission wavelength: 460 nm.
④ First, PO-PRO-1 Iodide (trade name; No. P-3581, manufactured by Molecular Probes) was added thereto (final concentration: 2.5 $\mu$M) whereupon Fd' was measured. Thereafter, Triton X-100 (trade name; manufactured by Pierce Chemical) was added (final concentration: 0.05%) whereupon Ft' was measured. Cell viability (or survival ratio of cells) was calculated from the above equation and the results are shown in Table 1.
⑤ For comparison, cell viability was also determined under a microscope for each sample by a Trypan Blue staining technique and the results are shown in Table 1. The Trypan Blue staining data were nearly the same as those in Example 1. It has been found that the method according to the present invention is well applicable for determining the cell viability. It is also possible to conduct this measurement in an automated manner using an apparatus as shown in Fig. 1.

Table 1

| Cell Viability | | |
|---|---|---|
| | Method of this Invention | Staining with Trypan Blue |
| Sample under good culture condition | 99.0% | 98.3% |
| Sample (1) under bad culture condition | 96.0% | 95.9% |
| Sample (2) under bad culture condition | 88.5% | 84.8% |

Example 2 (Quantitative Nature of Reaction between Cells and Stains for Nucleic Acid)

(1) Method and Result

① MOLT-4, HL-60 (human myelomonocytic leukemia cells), U937 (human histiocytic lymphoma cells) and HT-1080 (human fibrosarcoma cells) were used as test samples. All of the cell lines other than MOLT-4 were purchased from Dainippon Pharmaceutical Co., Ltd., Japan.
② The test samples were diluted 1:1 with a culture medium in terms of cell concentration to give diluted cell suspensions. The diluted cell suspension was plated in a 96-well microplate at 180 $\mu$l per well.

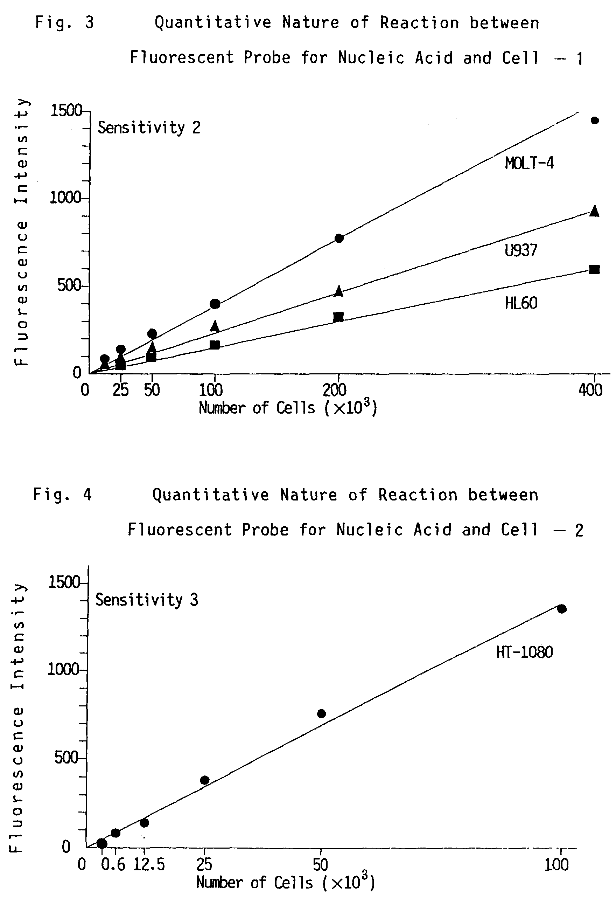

③ To each well was added 10 μl of PO-PRO-1 Iodide (trade name) (final concentration: 2.5 μM), followed by addition of 10 μl of Triton X-100 (trade name) (final concentration: 0.05%). The mixture was agitated for about 30 seconds using a plate mixer, followed by fluorescence measurement. The results are shown in Fig. 3 and Fig. 4.

Fig. 3 shows that there is a proportional relationship between the number of floating cells and the fluorescence intensity. For HL-60 and U-937, there is a proportional relationship between the number of floating cells and the fluorescence intensity when cell numbers are within a range from $1.25 \times 10^4$ to $4 \times 10^5$ while, for MOLT-4, there is a proportional relationship between the number of floating cells and the fluorescence intensity when cell numbers are within a range from $1.25 \times 10^4$ to $2 \times 10^5$

Fig. 4 shows that there is a proportional relationship between the number of adhesive cells and the fluorescence intensity. For HT-1080, there is a proportional relationship between the number of adhesive cells and the fluorescence intensity when cell numbers are within a range from 300 to $1 \times 10^5$.

Fluorescence intensity was measured with an MTP-32 Corona Microplate Reader (manufactured by Corona Denki K.K., Japan) at excitation wavelength: 420 nm and emission wavelength: 460 nm. This measurement can be also conducted in an automated manner using an apparatus as shown in Fig. 2.

(2) Discussion and Conclusion

From the results as shown in Figs. 3 and 4, it has been found that, although floating cells and adhesive cells are to be subjected to a fluorescence intensity measurement at different measuring ranges, there is a proportional relationship between the number of cells and the fluorescence intensity. As a result, viable cell numbers can be correctly determined by the method of the present invention.

<u>Example 3</u> (Evaluation of Cytotoxicity by Drugs)

(1) Method and Result

① Mitomycin C (anticancer agent; manufactured by Kyowa Hakko Kogyo Co., Ltd., Japan) was diluted with a culture medium. The diluted drug was distributed into a 96-well microplate at 50 μl per well.

② Thereafter, MOLT-4 cells were plated in an amount of $3 \times 10^4$ cells/150 μl per well.

③ Each well was incubated at 37°C for two days under a 100% humidified atmosphere of air containing 5% carbon dioxide and subjected to the measurement described hereinbelow. In a fluorescence measurement, an MTP-32 fluorometer (manufactured by Corona Denki K.K., Japan) was used.

④ To each well was added 10 μl of PO-PRO-1 Iodide (trade name) (final concentration: 2.5 μM), the mixture was agitated for about 30 seconds using a plate mixer and fluorescence was measured to determine Fd'.

⑤ To each well was further added 10 μl of Triton X-100 (trade name) (final concentration: 0.05%), the mixture was agitated for about 30 seconds using a plate mixer and fluorescence was measured to determine Ft'.

⑥ Viable cell numbers and cell viability were calculated from the aforementioned equation and the results are shown in Table 2.

⑦ For comparison, each sample was subjected to a viable cell number determination by an MTT assay system by referring to Cancer Research, Vol. 47, 936-942 (1987) and the results are shown in Table 2.

Further, steps for determination of viable cell numbers by the method according to the present invention and by the MTT assay are summarized in Table 3.

Similarly, the above-mentioned determination according to the present invention can be conducted in an automated manner using an apparatus as shown in Fig. 2.

Table 2

| Cytotoxicity Assessment of Mitomycin C | | | | | | |
|---|---|---|---|---|---|---|
| Concentration of Mitomycin C Added($\mu$g/ml) | | | | | | IC50 ($\mu$g/ml) |
| | 100 | 10 | 1 | 0.1 | 0 | |
| (Method of the Present Invention) | | | | | | |
| Viable cell numbers (% control) | 3 | 24 | 63 | 76 | 100 | 2.2 |
| Cell viability (%) | 29 | 87 | 100 | 99 | 98 | |
| (MTT Assay System) | | | | | | |
| Viable cell numbers (% control) | 3 | 34 | 95 | 120 | 100 | 5.5 |

Table 3

| Summary of Determination Steps for Viable Cell Number (Method of the Present Invention) | |
|---|---|
| ① PO-PRO-1 (50 $\mu$M): 10 $\mu$l/well | 1 minute |
| ② agitation with a microplate mixer | 30 seconds |
| ③ measurement of fluorescence at 420/460 | 30 seconds |
| ④ Triton X-100 (1%): 10 $\mu$l/well | 1 minute |
| ⑤ agitation with a microplate mixer | 30 seconds |
| ⑥ measurement of fluorescence at 420/460 | 30 seconds |
| Total time required | 4 minutes |
| (MTT Assay System) | |
| ① centrifugation at 2,000 rpm | 5 minutes |
| ② removal of supernatant liquid | 2 minutes |
| ③ MTT (0.04%): 100 $\mu$l/well | 1 minute |
| ④ incubation at 37°C, 5% $CO_2$-air | 3 hours |
| ⑤ PBS: 100 $\mu$l/well | 1 minute |
| ⑥ centrifugation at 2,000 rpm | 5 minutes |
| ⑦ removal of supernatant liquid | 2 minutes |
| ⑧ DMSO (100%): 150 $\mu$l/well | 1 minute |
| ⑨ agitation with a microplate mixer | 30 seconds |
| ⑩ measurement of absorbance at 550nm | 30 seconds |
| Total time required | 3 hrs and 20 min |

(2) Discussion and Conclusion

As shown in Table 2, the cytotoxicity of mitomycin C was estimated by the method of the present invention to be IC50 = 2.2 $\mu$g/ml. When this value is compared with that (5.5 $\mu$g/ml) in the estimation by an MTT assay system which has been most commonly used for evaluation of drug cytotoxicity, it is within an order of $\mu$g/ml and has a difference which is permissible in the related art. It is therefore clear that the method according to the present invention can be well applied for assessment of drug cytotoxicity. In addition, the method of the present invention has advantages that cell viabilities at various concentrations of drugs can be calculated simultaneously and accordingly that many information for evaluation of the drug is available. With regard to the time required for the determination, the method of the present invention finishes within about five minutes as shown in Table 3 and, as compared with the known MTT assay system (which requires three hours and twenty minutes), it is now possible to reduce the necessary time significantly. Further, in view of the operability, the method of the present invention does not need centrifugation, washing operation and dissolution of the dye. The instant technique is thus much more simple and convenient.

Example 4 (Damage to Cell Membrane by Ultrasonic Wave)

(1) Method and Result

① A cultured medium (40 ml) containing $5 \times 10^6$ MOLT-4 cells was used as a test sample, distributed into a cuvette for test sample and treated for 10 seconds with an ultrasonic pulse mastax (Sonicator Ultrasonic Processor Model W-225, Serial No. G6453, 20 kHz, Heat Systems-Ultrasonic Inc.) (Disruption intensity level: 3).
② The ultrasonicated sample in each cuvette was subjected to the measurement described hereinbelow while agitating with a stirring rod.
Fluorescence intensity was measured using a spectrofluorophotometer (Model F-4000; Hitachi, Ltd., Japan) at excitation wavelength: 420 nm and emission wavelength: 460 nm.
④ First, PO-PRO-1 lodide (trade name; No. P-3581, manufactured by Molecular Probes) was added thereto (final concentration: 2.5 μM). Fluorescence intensity was measured and found to be 410 (background fluorescence intensity: 10).

APPLICABILITY IN INDUSTRY

In accordance with the present invention, fluorescent probes for nucleic acid which stain dead cells only (or capable of exclusively staining dead cells) and agents for damaging cell membranes (or capable of damaging cell membranes) are used whereby viable cell numbers and/or cell viability of many samples can be determined conveniently and quickly and, furthermore, correctly. Accordingly, the present invention can be applied to cytotoxicity evaluation for pharmaceutical drugs, agricultural chemicals, cosmetics, foods, etc. In addition, the cytotoxicity assessment can be conducted conveniently and quickly as well.

**Claims**

1. A method for determining viable cell number and/or cell viability, which comprises

   (i) measuring the fluorescence intensity of a test sample subjected to a treatment with a fluorescent probe for nucleic acid, capable of exclusively staining dead cells,
   (ii) measuring the fluorescence intensity of a test sample subjected to not only (a) a treatment with said fluorescent probe for nucleic acid but also (b) a treatment of damaging cell membranes, and
   (iii) comparing both fluorescence intensities.

2. The method according to claim 1 wherein

   (i) the test sample is treated with a fluorescent probe for nucleic acid capable of exclusively staining dead cells, and the resultant fluorescence intensity is measured, and
   (ii) said test sample is then subjected to a treatment for damaging cell membranes with the result that cells are destined to become extinct, thereby intensifying the fluorescence thereof and the resultant fluorescence intensity thereof is measured.

3. The method according to claim 1 wherein

   (i) the test sample is treated with a fluorescent probe for nucleic acid capable of exclusively staining dead cells, and the resultant fluorescence intensity of said test sample is measured, and
   (ii) another test sample is subjected to not only (a) a treatment with said fluorescent probe for nucleic acid but also (b) a treatment of damaging cell membrane, and the resultant fluorescence intensity thereof is measured.

4. The method according to any of claims 1 to 3 wherein the fluorescent probe for nucleic acid is a cationic fluorescent stain or dye for nucleic acid.

5. The method according to any of claims 1 to 4 wherein the fluorescent probe for nucleic acid is ethidium homodimer or a cyanine fluorescent dye capable of exclusively staining dead cells.

6.  The method according to any of claims 1 to 5 wherein the cell membrane is damaged with an agent capable of damaging cell membranes.

7.  The method according to claim 6 wherein the agent capable of damaging cell membranes is a surface-active agent.

8.  The method according to claim 6 or 7 wherein the agent capable of damaging cell membranes is a polyoxyethylene nonionic surface-active agent or alcohol nonionic surface-active agent.

9.  The method according to any of claims 1 to 8 wherein each operation in a series of the following steps is conducted by an automated apparatus:

    (i) measuring the fluorescence intensity of a test sample subjected to a treatment with a fluorescent probe for nucleic acid, capable of exclusively staining dead cells,
    (ii) measuring the fluorescence intensity of a test sample subjected to not only (a) a treatment with said fluorescent probe for nucleic acid but also (b) a treatment of damaging cell membranes, and
    (iii) comparing both fluorescence intensities.

10. A kit for determining viable cell number and/or cell viability by the following steps:

    (i) measuring the fluorescence intensity of a test sample subjected to a treatment with a fluorescent probe for nucleic acid, capable of exclusively staining dead cells,
    (ii) measuring the fluorescence intensity of a test sample subjected to treatments with (a) said fluorescent probe for nucleic acid and (b) an agent capable of damaging cell membranes, and
    (iii) comparing both fluorescence intensities, which comprises

        (A) said fluorescent probe for nucleic acid, capable of exclusively staining dead cells, and
        (B) said agent capable of damaging cell membranes.

11. An apparatus for determining viable cell number and/or cell viability, which comprises at least

    (a) a means for supplying a test sample with a fluorescent probe for nucleic acid, capable of exclusively staining dead cells,
    (b) a means for measuring the fluorescence intensity of the test sample which is treated with said fluorescent probe for nucleic acid,
    (c) a means enabling us to damage cell membrane,
    (d) a means for measuring the fluorescence intensity of the test sample which is subjected to not only (A) a treatment with said fluorescent probe for nucleic acid but also (B) a treatment of damaging cell membrane, and
    (e) a means for comparing the fluorescence intensity (b) with that (d).

12. The apparatus according to claim 11 wherein said apparatus is equipped with:

    (a) a device for holding an agent capable of damaging cell membranes,
    (b) a device for holding a fluorescent probe for nucleic acid capable of exclusively staining dead cells,
    (c) an injector for drug capable of supplying the test sample with the agent from the above-mentioned device (a) or (b),
    (d) a container capable of holding the test sample therein, and
    (e) a spectrofluorophotometer.

Fig. 1    Apparatus for Determining Viable Cell Number

Fig. 2     Apparatus for Determining Viable Cell Number

Fig. 3    Quantitative Nature of Reaction between
Fluorescent Probe for Nucleic Acid and Cell — 1

Fig. 4    Quantitative Nature of Reaction between
Fluorescent Probe for Nucleic Acid and Cell — 2

## INTERNATIONAL SEARCH REPORT

| | International application No. |
|---|---|
| | PCT/JP96/03842 |

**A. CLASSIFICATION OF SUBJECT MATTER**

Int. Cl$^6$  G01N33/48, C12Q1/00

According to International Patent Classification (IPC) or to both national classification and IPC

**B. FIELDS SEARCHED**

Minimum documentation searched (classification system followed by classification symbols)

Int. Cl$^6$  G01N33/48, C12Q1/00

Documentation searched other than minimum documentation to the extent that such documents are included in the fields searched

| | |
|---|---|
| Jitsuyo Shinan Koho | 1926 - 1996 |
| Kokai Jitsuyo Shinan Koho | 1971 - 1996 |
| Toroku Jitsuyo Shinan Koho | 1994 - 1996 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 02-117397, A (Mitsubishi Heavy Industries, Ltd.), May 1, 1990 (01. 05. 90)(Family: none) | 1 - 20 |
| A | US, 5321130, A (Molecular Probes, Inc.), June 14, 1994 (14. 06. 94)(Family: none) | 4, 5 |

☐ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| April 1, 1997 (01. 04. 97) | April 15, 1997 (15. 04. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)